## Europäisches Patentamt
(19) **European Patent Office**
Office européen des brevets

(11) Publication number: **0 266 323**
**A1**

(12) # EUROPEAN PATENT APPLICATION

(21) Application number: **87850283.0**

(22) Date of filing: **18.09.87**

(51) Int. Cl.⁴: **A 23 D 5/00**
**A 23 D 5/02, A 61 K 31/23**

(30) Priority: **29.09.86 SE 8604117**

(43) Date of publication of application:
**04.05.88 Bulletin 88/18**

(84) Designated Contracting States: **ES GR**

(71) Applicant: **KabiVitrum AB**
**Lindhagensgatan 133**
**S-112 87 Stockholm (SE)**

(72) Inventor: **Vogeler, Jan Ingemar**
**Granitvägen 43A**
**S-183 63 Täby (SE)**

**Andersson, Nils-Erik Lennart**
**Vallstavägen 81**
**S-190 40 Rosersberg (SE)**

(74) Representative: **Hjertman, Ivan T. et al**
**AB ASTRA Patent and Trade Mark Department**
**S-151 85 Södertälje (SE)**

(54) **Granulate containing gamma-linolenic acid, eicosa pentaenoic acid and/or docosahexaenoic acid, method for its manufacturing, its use in edible products, and a tablet containing it.**

(57) A granulate comprising an oil-powder mixture containing 2-75 % vegetable oil and/or marine oil containing essential fatty acids selected from gamma linolenic acid (GLA), eicosapentaenoic acid (EPA) and/or docosahexanenoic acid (DHA) and/or other marine oils and a water-soluble carrier, in combination with a solid pulverulent filler and a binder. Tablets containing said granulate. Use of said granulate and tablets in pharmacy, dietary supplements, food products and fooder.

**Description**

Composition

Field of the invention

The present invention relates to a granulate comprising an oil-powder mixture containing 2-75 % oil concentration of vegetable and/or marine oils and a water-soluble carrier in combination with a solid pulverulent filler and a binder, and to the preparation of said granulate, use of said granulate especially for the preparation of tablets, as well as said tablets.

Background of the invention

Currently available vegetable and marine oils containing essential fatty acids are administrated in fluid form in bottles, containers or in soft or hard gelatin capsules. Particularly, this applies to oils containing gamma linolenic acid (GLA), eicosapentaenoic acid (EPA) or docosahexaenoic acid (DHA).

A great disadvantage with these oils in oil phase is that they can not be mixed with water-soluble substances.

A long-felt want is to prepare tablets comprising a high content of the essential fatty acids GLA, and/or EPA/DHA, since tablets are an administration form easy of access. However, a lot of practical and technical problems have prevented the preparation of such tablets. Surprisingly, the preparation of a granulate according to the present invention has been capable of solving these problems.

Description of the invention

The object of the present invention is to prepare a granulate, having a high content of fatty acids and compressability properties especially suitable for the preparation of tablets for application in pharmacy, dietary supplements, food products and veterinary medicine.

Oils suitable for conversion to an oil powder mixture are vegetable oils and marine oils containing essential fatty acid, especially oils containing gamma linolenic acid (GLA), eicosapentaenoic acid (EPA) and/or docosahexaenoic acid (DHA). Examples of oils suitable in mixed or unmixed form to be convertible to powder phase are vegetable oil such as black currant oil, borage oil, evening primrose oil, wheat germ oil (vitamin E), biosynthetic gamma linolenic acid oil and marine oils such as cod liver oil, salmon oil and shark liver oil.

Antioxidant e.g. vitamin E (alpha tocopherol) or combined alpha tocopherol and ascorbyl palmitate is added to the oil for protection against oxidation of the granulate in the manufacture and storing.

Carriers suitable for mixing with the oil to convert the oil to an oil powder are a defatted, water-soluble carrier alone or in combination. Such carriers are proteins such as plant proteins e.g. soybean protein, animal proteins e.g. egg protein, milk protein, caseinate, polysaccharides such as starches e.g. corn starch, potato starch, amylopectin or cellulose.

By a careful choice of vegetable carriers and vegetable oils, an oil powder mixture free from animal substances can be prepared, which is important in manufacture of several products for allergical persons.

Suitable solid, pulverulent fillers for mixing with the oil-powder mixture are sugars or modified sugar products e.g. dextrose, lactose, fructose, saccharose, sorbitol, mannitol, potato starch, corn starch, amylopectin or cellulose.

Conventional binders are such as unmodified or modified polysaccharide products, for instance cellulose polymers such as methyl cellulose; hydroxy propyl starch or acacia (Arab.gum).

Examples of additives are vitamins, enzymes, minerals, pollen products, ginseng products, flavouring agents and pharmaceutically active substances such as antibiotics, hormones, cortisones etc.

The preparation of the oil-powder mixture is in the following way:

(i) heating the oil to e.g. 20-70°C depending on the oil used; optionally adding of an antioxidant a monoglyceride preferably 0,1-1,0 % (monoglyceride w/oil w) vegetable monoglyceride with vigorous stirring. The monoglyceride makes the homogenisation easier.

(ii) simultanously, a defatted carrier is dissolved in water with vigorous stirring and heating from e.g. 20°C to 65°C depending on the carrier used,

(iii) the mixture (i) containing the oil and the mixture (ii) containing the carrier in water solution are mixed with vigorous stirring,

(iv) the resulting mixture is then emulsified by means of a conventional high pressure homogenizer working at a pressure above 100 kg/cm$^2$ (100-250 kg/cm$^2$),

(v) the emulsion formed is dried in a conventional spray-drier. The emulsion out-coming from the nozzle meet warm air having a temperature about 200°C. The powder formed in the spray-drier has a temperature of about 70°C, which can be lowered to room temperature (18-23°C) by passing a fluid-bed drier or similar device.

The oil-powder mixture has an average particle size of from 0,1 to 1,5μ, at agglomeration the particle size increases. The oil concentration of the oil-powder mixture may vary from 2 % to 75 % especially from 20 % to 75 % (w/w) preferably between 40 % to 75 % (w/w) depending on the amount of oil being added to the carrier solution (step iii above).

The preparation of the granulate of the present invention is performed in the following way:

2

(a) dry-mixing the oil-powder mixture, optionally containing an antioxidant, with a solid, pulverulent filler preferably dextrose, optionally adding of a diluent, with vigorous stirring,

(b) simultanously, a conventional binder e.g. methyl cellulose, hydroxy propyl starch is dissolved in water with vigorous stirring,

(c) the dry-mixture (a) containing the oil-powder mixture and the filler and the mixture (b) containing the dissolved binder are mixed with vigorous stirring,

(d) the granulate obtained is then dried preferably by means of a microwave oven at a temperature between 20-50° C.

(e) the dried granulate is then driven optionally together with additives through a sieve to desired size.

The granulate has preferably a particle size of from 0.5 to 3.0 mm

Drying of the granulate in microwave oven gives a very short drying time and prevent oxidation.

The advantage of the granulate according to the invention is:
- that the granulate has a high content of oils
- that the granulate has suitable compressability properties to admit in the preparation of tablets, for human and animal use
- that the granulate can be mixed with other dry substances which means increased field of use, such as mixing in food products, in fodder, dietary supplement, pharmaceuticals etc.

In preparation of compositions containing essential fatty acids in the form of dosage units for oral administration tablets are a preferred administration form.

Tablets are a preferred administration form compared to capsules since the dosage of the active substance can be varied within a much wider range compared with capsules. It is easy to make a tablet of a suitable size containing a sufficient amount of active substance. Several people prefer tablets to capsules owing to taste, design, easier to swallow etc.

Tablets of the present invention are especially suitable as chewing tablets or effervescent tablets. The chewing tablets may also include vegetable fibres. Incorporation of an acid/bicarbonate mixture e.g. a tartaric acid/sodium bicarbonate mixture, make the tablets effervescent on addition of water.

The tablets are prepared by pressing the granulate obtained optionally with addition of additives in a conventional tableting machine.

Additives can optionally be added in the preparation of the granulate and/or in preparation of the tablets.

The invention will be illustrated by the following examples without being limited thereto.

Example 1 Oil-powder mixture, 50 % borage oil

Components
Soybean protein     480 kg
Borage oil     480 kg
Water     1 760 1
Vegetable monoglyceride     1 kg     Soybean protein was dissolved in water with vigorous stirring and heating to about 65°C. Borage oil containing antioxidant was heated to 65°-70°C and monoglyceride was added with vigorous stirring. The borage oil was mixed with the aqueous soybean protein solution with vigorous stirring. Then the mixture obtained was pumped to a conventional homogenizer working at a pressure above 100 $kg/cm^2$ (100-250 $kg/cm^2$). The emulsion formed was then dried in a spray-drier, wherein the warm air has a temperature of 200°C. The outcoming oil-powder mixture has a temperature of 70°C, which temperature was lowered to 20°C by passing a fluid-bed drier. The resulting oil-powder mixture containing 50 % (w/w) oil has an average particle size of from 0,1 to 0,5 μ.

Example 2 Oil-powder mixture, 50 % marine oil

Components
Soybean protein     480 kg
Marine oil     480 kg
Water     1 760 1
Vegetable monoglyceride     3 kg     The oil-powder mixture was prepared in the same way as described in Example 1.

Example 3 GLA granulate

Components
Borage oil-powder mixture (50 % oil)     350 mg
Dextrose (EMDEXR )     250 mg
Methyl cellulose     15 mg     The components were mixed to a granulate in the following way:
10 mg Methyl cellulose was mixed with water and the mixture was stored for 1-1,5 hours. The borage oil-powder mixture as prepared in Example 1 was dry-mixed with dextrose and 5 mg methyl cellulose. The methyl cellulose was added as a binder. Thereafter the dry-mixture was granulated with the water solution and the granulate obtained was dried in an oven during 24 hours or in a microwave oven, alternatively. After drying the granulate was driven through a sieve to desired size. The granulate obtained may thereafter be pressed to a tablet in a conventional tableting machine with addition of a conventional binder such as a cellulose derivative e.g. AvicelR .

### Example 4 GLA granulate

Components
Borage oil-powder mixture (50% oil)     350 mg
Dextrose (EMDEXR)     250 mg
Hydroxy propyl starch     100 mg     The components were mixed to a granulate in the same way as described in Example 3.

### Example 5 GLA-Vitamin B6 granulate

Components
Borage oil-powder mixture (50% oil)     350 mg
Dextrose (EMDEXR)     250 mg
Hydroxy propyl starch     100 mg
Pyridoxine chloride     20 mg     The components were mixed to a granulate in the same way as described in Example 3.

### Example 6 EPA/DHA granulate

Components
Cod liver oil-powder mixture (50 % oil)     350 mg
Dextrose (EMDEXR)     250 mg
Methyl cellulose     15 mg     The components were mixed to a granulate in the same way as described in Example 3.

### Example 7 GLA/EPA/DHA granulate

Components
Borage oil-powder mixture (50 % oil)     250 mg
Shark liver oil-powder mixture (50 % oil)     150 mg
Dextrose     300 mg
Methyl cellulose     20 mg     The components were mixed to a granulate in the same way as described in Example 3.

The ratio by weight of GLA oil-powder mixture and EPA/DHA oil-powder mixture in the combination granulate may vary.

### Example 8 GLA-pollen-lecithin tablet

| Composition | Tablet content |
| --- | --- |
| GLA granulate | 700 mg |
| Pollen concentrate | 95 mg |
| Soybean lecithin | 95 mg |
| Cellulose (Avicel$^R$) | 100 mg |
| Average weight | 990 mg |

The granulate obtained in Example 4 was dry-mixed with the components pollen concentrate and lecithin and the binder AvicelR with stirring and then pressed to tablets in a conventional tableting machine.

Example 9  GLA-selenium tablet

| Composition | Tablet content |
|---|---|
| GLA granulate | 615 mg |
| Sodium selenite | 0.05 mg |
| Cellulose (Avicel$^R$) | 100 mg |
| Average weight | 715 mg |

The granulate obtained in Example 3 was mixed with the other components and formed to tablets in the same way as described in Example 8.

Example 10  GLA-vitamin B6 tablet

| Composition | Tablet content |
|---|---|
| GLA granulate | 700 mg |
| Pyridoxine chloride | 20 mg |
| Cellulose Avicel$^R$ | 100 mg |
| Fructose | 40 mg |
| Citric acid | 12 mg |
| Arom. lemon | 28 mg |
| Average weight | 900 mg |

The granulate obtained in Example 4 was mixed with the other components and formed to tablets in the same way as described in Example 8.

Granulate containing GLA and tablets containing GLA can be prepared from black currant oil, evening primrose oil, wheat germ oil or biosynthesis optionally with additives in the same way as described in Examples 3-5 and 7-10.

Granulate and tablets containing EPA/DHA can be prepared from marine oils optionally with additives in the same way as described in Examples 6-10.

**Claims**

1. Granulate comprising
(i) an oil-powder mixture containing 2-75 % (w/w) vegetable oil and/or marine oil, wherein the essential fatty acids are selected from gamma-linolenic acid (GLA), eicosapentaenoic acid (EPA) and/or docosahexanenoic acid (DHA), and/or other marine oils and a water-soluble carrier in combination with
(ii) a solid, pulverulent filler and
(iii) a binder.

2. Granulate comprising
(i) an oil-powder mixture containing 2-75 % (w/w) vegetable oil and/or marine oil, wherein the essential fatty acids are selected from gamma-linolenic acid (GLA), eicosapentaenoic acid (EPA) and/or docosahexanenoic acid (DHA), and/or other marine oils and a water-soluble carrier, in combination with

(ii) a solid, pulverulent filler and

(iii) a binder, said granulate is obtainable by

a) dry-mixing the oil-powder mixture optionally containing an antioxidant with the solid, pulverulent filler and optionally adding a diluent etc.,

b) dissolving a binder in water,

c) mixing the dry-mixture (a) and the dissolved binder (b) with vigorous stirring, to obtain a granulate,

d) drying the granulate obtained and

e) driven the dried granulate, optionally together with additives to desired size.

3. Granulate according to claims 1 or 2 wherein the oil-powder mixture containing 40 to 75 % (w/w) oil.

4. Granulate according to any of claims 1-3 wherein the oil-powder mixture containing 50 % (w/w) oil.

5. Granulate according to any of claims 1-4. wherein the oil is borage oil, cod liver oil or shark liver oil.

6. Granulate according to any of claims 1-5 wherein the water-soluble carrier is soybean protein.

7. Granulate according to any of claims 1 or 2 wherein the filler is a sugar or a modified sugar e.g. dextrose.

8. Granulate according to any of claims 1 or 2 wherein the binder is a polysaccharide or modified polysaccharide e.g. methyl cellulose, hydroxy propyl starch.

9. Granulate comprising an oil-powder mixture containing 50 % (w/w) borage oil and soybean protein in combination with dextrose and methyl cellulose or hydroxy propyl starch.

10. Granulate according to any of claims 1-9 containing an additive such as vitamins, minerals, antibiotics, hormones, cortisones, pollen products and ginseng products.

11. A method for manufacturing

(i) a granulate comprising an oil-powder mixture containing 2-75 % (w/w) vegetable oil and/or marine oil, wherein the essential fatty acids are selected from gamma-linolenic acid (GLA), eicosapentaenoic acid (EPA) and/or docosahexanenoic acid (DHA), and/or other marine oils and a water-soluble carrier, in combination with

(ii) a solid, pulverulent filler and

(iii) a binder characterized by

a) dry-mixing the oil-powder mixture optionally containing an antioxidant with the solid, pulverulent filler and optionally adding a diluent, etc.,

b) dissolving a binder in water,

c) mixing the dry-mixture (a) and the dissolved binder (b) with vigorous stirring, to obtain a granulate

d) drying the granulate obtained and

e) driven the dried granulate optionally together with additives to desired size.

12. Use of a granulate according to any of claims 1-10 in dietary supplement products, in food products fodder or in pharmaceutical products.

13. Use of a granulate according to any of claims 1-10 for the manufacture of tablets.

14. Tablet comprising a granulate according to any of claims 1-10 in combination with a binder.

15. Tablet according to claim 14 containing an acid/bicarbonate mixture.

16. Tablet according to any of claims 14-15 containing additives such as vitamins, minerals, antibiotics, enzymes, hormones, cortisones, pollen products and ginseng products.

17. Tablet according to claim 16 wherein the additive is vitamin B6.

18. Tablet according to claim 16 wherein the additive is physiologically assimilable selenium.

19. Tablet according to claim 16 wherein the additive is lecithin and pollen products.

20. Use of a tablet according to any of claims 14-19 as a pharmaceutical product or as a dietary supplement product.

European Patent Office

**PARTIAL EUROPEAN SEARCH REPORT**
which under Rule 45 of the European Patent Convention
shall be considered, for the purposes of subsequent
proceedings, as the European search report

Application number

EP 87 85 0283.0

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X<br>Y | US-A-4 564 475 (MASAICHIRO)<br>January 14, 1986<br>*Col 3 line 27-46* | 1,12-14<br>2-10,<br>15-20 | A 23 D 5/00,02<br>A 61 K 31/23 |
| | & GB 2 140 806<br>DE 3 419 796<br>JP 59219396<br>FR 2 547 829<br>JP 600113738 | | |
| | --- | | |
| X<br>Y | EP-A3-0 092 076 (SOCIETE DES PRO-<br>DUITS NESTLE S A)<br>October 26, 1983<br>*Example 6*<br>& EP 0 092 085<br>GB 2 118 567<br>JP 58189110<br>AU 13084/83<br>AU 13169/83<br>JP 58192828<br>CA 1 195 172 | 1,12-14<br>2-10,<br>15-20 | |
| | --- | | TECHNICAL FIELDS SEARCHED (Int. Cl.4) |
| Y | EP-A3-0 175 468 (SENTRACHEM LIMITED)<br>March 26, 1986<br>*Claim 7 and 8 page 3*<br>-/- | 1-20 | A 23 D<br>A 61 K |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-19

Claims searched incompletely: 20*

Claims not searched:

Reason for the limitation of the search:

*) (to the extent that terapeutical treatment is concerned)
Method for treatment of the human or animal body by therapy (see art 52(4) of the European Patent Concention.

| Place of search | Date of completion of the search | Examiner |
|---|---|---|
| STOCKHOLM | 1-12-1987 | BOTJE JANSON K. |

BAD ORIGINAL

## DOCUMENTS CONSIDERED TO BE RELEVANT

| Category | Citation of document with indication, where appropriate, of relevant passages | Relevant to claim |
|---|---|---|
| | & AU 45959/85<br>JP 61083122<br><br>--- | |
| Y | EP-A1-0 012 499 (R G B LABORATORIES, INC)<br>June 25, 1980<br>*Page 3, line 2-9*<br>& US 4 242 364<br>   AU 51464/79<br>   JP 55085357<br>   CA 1 119 454<br>   AU 528097<br><br>--- | 2,11 |
| A | GB-A- 1 580 444 (BIO-OIL RESEARCH LIMITED)<br>December 3, 1980<br><br>----- | 1-20 |

**CLASSIFICATION OF THE APPLICATION (Int. Cl.4)**

**TECHNICAL FIELDS SEARCHED (Int. Cl.4)**

EPO Form 1505.3   06.78